# EUROPÄISCHE PATENTANMELDUNG

(11) **EP 2 604 246 A1**
(43) Veröffentlichungstag der Anmeldung: **19.06.2013**
(21) Anmeldenummer: 11009892.8
(22) Anmeldetag: 15.12.2011
(51) Int. Cl.: A61K 6/00

(54) **Verfahren zum Einfärben von Dentalkeramiken**

(71) Anmelder: STEGER, Heinrich, 39031 Bruneck (IT)
(72) Erfinder: STEGER, Heinrich, 39031 Bruneck (IT)
(74) Vertreter: Gangl, Markus

(57) **Zusammenfassung**

Die vorliegende Erfindung betrifft ein Verfahren zum Einfärben von Dentalkeramiken, ein dentalkeramisches, metallhaltiges Färbemittel sowie eine Dentalkeramik, wobei das dentalkeramische metallhaltige Färbemittel als Trägersubstanz ein Gel aufweist und als Gel auf die Dentalkeramik aufgebracht wird bzw. ist.

## Beschreibung

Die vorliegende Erfindung betrifft ein Verfahren zum Einfärben von Dentalkeramiken gemäß dem Oberbegriff des Anspruchs 1, ein dentalkeramisches Färbemittel gemäß dem Oberbegriff des Anspruchs 2, und eine Dentalkeramik gemäß dem Oberbegriff des Anspruchs 3.

Aus der DE 199 04 522 B4 geht ein Verfahren zum Einfärben von keramischem Zahnersatz gemäß dem Oberbegriff des Anspruchs 1 hervor. Problematisch an diesem Verfahren ist die Tatsache, dass die dort als Lösungen eingesetzten Färbemittel eine Reihe von Nachteilen aufweisen, wie beispielsweise die schlechte Dosierbarkeit, schlechte Transportfähigkeit usw.

Aufgabe der Erfindung ist es ein Verfahren, ein dentalkeramisches Färbemittel sowie eine Dentalkeramik bereitzustellen, bei denen diese Nachteile nicht auftreten.

Diese Aufgabe wird durch ein Verfahren mit den Merkmalen des Anspruchs 1, ein dentalkeramisches Färbemittel mit den Merkmalen des Anspruchs 2 sowie eine Dentalkeramik mit den Merkmalen des Anspruchs 3 gelöst. Vorteilhafte Ausführungsformen der Erfindung gehen aus den abhängigen Ansprüchen hervor.

Unter dem bei der Erfindung eingesetzten Gel versteht man ein feindisperses System aus mindestens zwei Phasen, nämlich im Falle der Erfindung einer flüssigen und einer festen Phase, bei denen die feste Phase als zusammenhängendes dreidimensionales Gerüst vorliegt, in dem die flüssige Phase eingeschlossen ist. Beide Phasen durchdringen sich vollständig, das heißt sie sind bikohärent.

Die Vorteile des Einsatzes eines Färbemittels in Form eines Gels gegenüber einer Lösung gemäß dem Stand der Technik sind vielfältig:
- je nachdem wie viel Flüssigkeit zugegeben wird, ist es möglich verschieden viskose Gele zu erzeugen, von einer öligen bis hin zu einer cremartigen Konsistenz,
- nach dem Dichtsintem des eingefärbten Keramikobjektes weist dies eine homogenere Einfärbung an der Oberfläche auf,
- man kann das gelartig ausgebildete Färbemittel besser dosieren und auch auf der Dentalkeramik besser platzieren (dieses verläuft weniger als eine Lösung),
- es gibt Vorteile beim Transport gegenüber dem Einsatz einer Lösung (kein Auslaufen, kein Verschütten, kein Aufblähen der Behälter bei zu langer Wärmeinwirkung),
- es ist möglich, das gelartige dentalkeramische Färbemittel maschinell aufzutragen (zum Beispiel zu Verspritzen) ohne einen Wassernebel zu erzeugen. Dies bewirkt, dass die Elektronik der Maschine nicht wasserdicht sein muss.

Die Dentalkeramik weist vorzugsweise als keramischen Hauptbestandteil Zirkonoxid (ZrO₂) auf.

Zur Herstellung des Gels wird Gelbildner, vorzugsweise Zellulose, zum Beispiel Hydroxyethylcellulose (HEC) eingesetzt. Ferner können auch andere Gelbildner verwendet werden, zum Beispiel Silicagel, Polyacrylsäure, Gelatine, Stärke. Vorzugsweise ist der Gelbildner ein organischer Gelbildner, wie die oben genannte Zellulose oder ein LMOG ("Low Molecular Mass Organic Gelator").

Das Gel kann durch entsprechend niedrige bzw. hohe Beimengung von Gelbildner zur Flüssigkeit (durch Versuche bestimmbar) auch mit öliger bzw. cremartiger Konsistenz ausgestaltet werden.

Als Metall/Metallverbindungen/Metallkomplexe werden vorzugsweise Metallsalze (insbesondere Metallnitrate) eingesetzt, die je nach Farbe ausgewählt werden. Es ist möglich, all jene Metalle/Metallverbindungen/Metallkomplexe einzusetzen, die in der DE 199 04 522 B4 angeführt sind, bevorzugt Eisen (Fe), Chrom (Cr), Mangan (Mn), Erbium (Er), Neodynium (Nd), Kobalt (Co), Bismuth (Bi) und Praseodymium (Pr).

Die Konzentration Metallionen (Kationen) sollte Bereich von 0,001 bis 3 Gew% liegen. Die Gegenionen (Anionen) können im Bereich von durchaus bis 20 Gew% liegen. Die Summe ergibt dann die Gesamtkonzentration im Färbemittel.

Ein Verfahren zur Herstellung einer eingefärbten Dentalkeramik unter Verwendung eines erfindungsgemäßen dentalkeramischen metallhaltigen Färbemittels kann wie folgt aussehen:
● Bereitstellen von pressfertigem Pulvermix aus Zirkoniumdioxid ZrO₂ (zum Beispiel Ytriumstabilisiertes Zirkoniumdioxid, ggf. inkl. Bindemittel)
● Pressen des Pulvers zu Blöcken (uniaxial oder isostatisch) => Grünling
● Entbindem der Blöcke
● Vorsintern der Blöcke (Zirkonpulver verschmilzt nur an den Kontaktstellen). Es entsteht ein poröser Zirkonrohling (= Weißling)
e Fräsen der Dentalkeramik (Gerüst, welches noch zu verblenden ist oder bereits endgültige Form des Zahnersatzes oder eine beliebige Kombination - teilweise zu verblenden und teilweise fertig)
● Aufbringen des Färbemittels auf die Dentalkeramik
● Aufgebrachtes Färbemittel trocknen
● Dentalkeramik dicht sintern (je nach Material von 1400°C bis 1600°C Endtemperatur). Aufheizrate kann je nach Größe und Gewicht des Gegenstandes von 3°C/min bis 50°C/min (gängig sind Aufheizraten von 8-14°C/min)
● Nach dem Sintern ist die Dentalkeramik evtl. noch etwas zu beschleifen und zu polieren.
● Es folgt eine evtl. Keramikschichtung

Die Herstellung des als Trägersubstanz dienenden Gels des erfindungsgemäßen dentalkeramischen metallhältigen Färbemittels kann beispielsweise wie folgt erfolgen:

Zellulose, beispielsweise Hydroxyethylcellulose wird in der gewünschten Konzentration (beispielsweise 2,5 Gew%) mit vorzugsweise Wasser oder einer anderen Flüssigkeit, beispielsweise Alkohol, abgemischt. Diese Mischung lässt man aufquellen, wodurch das Gel entsteht. Anschließend können Konservierungsstoffe und/oder ein Stabilisierungsmittel und/oder ein Malhilfsmittel beigemischt werden. Anschließend wird je nach gewünschter Farbe das wenigstens eine Metall bzw. die entsprechende Metallverbindung/Metallkomplexverbindung (zum Beispiel Metallsalze) dazugegeben.

Als Konservierungsmittel können zum Beispiel Nipagin (Methyl-4-hydroxybenzoat, das heißt C₁₇H₁₇Cl₂N_{3<}) und/oder Nipasol (Propyl-4-hydroxybenzoat, das heißt C₁₀H₁₂O₃) eingesetzt werden.

## Patentansprüche

1. Verfahren zum Einfärben von Dentalkeramiken, insbesondere Gerüstkeramiken oder Vollkeramiken, im porösen oder saugfähigen Zustand, mit einem metallhaltigen Färbemittel, wobei die Dentalkeramiken im entbinderten und/oder vorgesinterten Zustand eingefärbt werden, **dadurch gekennzeichnet, dass** das Färbemittel als Gel auf die Dentalkeramik aufgebracht wird.

2. Dentalkeramisches, metallhaltiges Färbemittel, umfassend:
- ein Gel als Trägersubstanz,
- wenigstens ein in das Gel eingebrachtes Metall, Metallverbindung oder Metallkomplexverbindung,
- gegebenenfalls ein Stabilisierungsmittel,
- gegebenenfalls ein Konservierungsmittel
- gegebenenfalls ein Malhilfsmittel.

3. Dentalkeramik, insbesondere Gerüstkeramik oder Vollkeramik, **dadurch gekennzeichnet, dass** die Dentalkeramik wenigstens bereichsweise mit einem dentalkeramischen Färbemittel nach Anspruch 2 eingefärbt ist.

4. Verfahren nach Anspruch 1 oder Dentalkeramik nach Anspruch 3, **dadurch gekennzeichnet, dass** das keramische Material als Hauptbestandteil Zirkoniumdioxid aufweist.

5. Färbemittel nach Anspruch 2, **dadurch gekennzeichnet, dass** das Gel beinhaltet:
- 0,1 - 10 Gew%, vorzugsweise 1 - 5 Gew%, besonders bevorzugt 2 - 5 Gew%, zum Beispiel ca. 2,5 Gew% Gelbildner, zum Beispiel Hydroxyethylcellulose oder Silicagel,
- 0 - 10 Gew%, vorzugsweise 4 - 6 Gew%, zum Beispiel ca. 5 Gew% Stabilisierungsmittel, zum Beispiel Polyethylenglykol,
- 0,01 - 1 Gew%, vorzugsweise 0, 04 - 0,3 Gew%, zum Beispiel ca. 0,14 Gew% Konservierungsmittel, zum Beispiel ca. 0,1 Gew% Nipagin und ca. 0,04 Gew% Nipasol,
- Rest Flüssigkeit - vorzugsweise Wasser oder Alkohol.

6. Färbemittel nach Anspruch 2, **dadurch gekennzeichnet, dass** das Gel mindestens ca. 2,5 Gew% Gelbildner, zum Beispiel Hydroxyethylcellulose oder Silicagel, beinhaltet.

7. Verfahren nach Anspruch 1 oder 4 oder Färbemittel nach Anspruch 2 oder 5, **dadurch gekennzeichnet, dass** das Gel mit öliger oder cremeartiger Konsistenz ausgebildet ist.
